# EUROPEAN PATENT SPECIFICATION

(11) **EP 0 314 499 B1**
(45) Date of publication and mention of the grant of the patent: **20.01.1993**
(21) Application number: 88310160.2
(22) Date of filing: 28.10.1988
(51) Int. Cl.: G01N 33/52, G01N 33/558, G01N 33/543

(54) **Devices and methods for chemical testing**
Vorrichtungen und Verfahren für chemische Untersuchungen
Dispositifs et procédés pour des essais chimiques

(30) Priority: 30.10.1987 GB 8725458
(43) Date of publication of application: 03.05.1989
(73) Proprietor: UNILEVER PLC, London EC4P 4BQ (GB); UNILEVER N.V., 3013 AL Rotterdam (NL)
(72) Inventor: May, Keith, Bromham Bedfordshire (GB); Prior, Michael Evans, Rushden Northamptonshire (GB)
(74) Representative: Butler, David John

(56) References cited:
- EP-A- 0 186 799
- EP-A- 0 239 174
- EP-A- 0 262 328

## Description

This invention relates to devices and methods for chemical testing, and especially devices and methods for microchemical testing of clinical sample materials, e.g. urine, blood, serum, etc.

Some recent examples of prior art devices and methods are shown by EP 0 100 619 and USP 4,435,504 (Syva) which concern immunochromatographic assays, which exploit the specific binding of a material relevant to the assay with a binding partner carried on a filter-paper support. Similarly, combinations of bibulous support materials with immunoassay methods are the subjects of USP 4,168,146 (Kabi), USP 4,461,829 (Miles), USP 4,517,288 (American Hospital Supply), and EP 186,799 (Behringwerke).

In particular, EP 186799 (Behringwerke) describes chromatographic test devices wherein a liquid stream containing a test sample and/or other reagents flows along a porous carrier material, and the test result is developed in a detection zone in which a specific binding reagent is immobilised. In one embodiment, EP 186799 suggests that more than one body of porous carrier material can be provided in the test device, and that delayed delivery of reagents associated with a signal-developing system can thereby be delivered to the detection zone via a liquid stream that flows parallel to the main liquid stream, but more slowly. In EP 186799 it is suggested that such a parallel liquid stream can be controlled by using a more slowly chromatographing absorbent medium such as a selected chromatographic paper, or a paper that has been impregnated at places with components that temporarily block the mobile phase, such as polymers that confer a high viscosity when dissolved.

The present invention is also concerned with test devices which incorporate a plurality of liquid-conductive zones which deliver separate liquid streams to a region in the test device during the assay procedure. Although it is possible to provide such a test device in which the separate liquid-conductive zones are in permanent contact at the point at which the separate liquid streams need to come into contact with one another, and hence control of the relative liquid flows is achieved for example by the use of conductive materials having different properties as suggested in EP 186799, we believe that much greater control can be achieved if at least one of the liquid flow paths within the device is enhanced or made and/or broken or restricted during the course of the test.

The present invention provides test devices having at least two liquid flow paths and wherein at least one of the flow paths incorporates a swellable "switch" which influences the delivery of material in that flow path to another flow path within the device. No such suggestion is found in EP 186799. EP 262328 discloses devices having multiple liquid flow pathways separately leading to a common zone, but there is no suggestion that relative liquid flow in such pathways should be modulated by means of a swellable material. EP 239174, which is concerned with devices having only one liquid flow pathway incorporating a test element, indicates that swelling of the test element may affect the rate of which fluid is delivered to it. However, this swelling is presented as a negative feature, and EP 239174 suggests ways in which the effects of any such swelling can be avoided.

The present invention provides a test device for detecting the presence of an analyte in a liquid sample and incorporating a plurality of liquid-conductive zones which form separate liquid flow paths which deliver separate liquid streams to a region in the test device during the test procedure, characterised in that the device incorporates a liquid-swellable material which is arranged to swell by contact with liquid sample and/or reagent and thereby to enhance or make or break or restrict contact between two liquid-conductive zones, during the course of the test procedure, to control relative liquid flows in the separate flow paths.

For example, a water-swellable or aqueous liquid-swellable material can be used to make or enhance contact between a first and second conductive zone and thereby initiate or promote transfer of a washing and/or reagent liquid between the zones. Such transfer can occur for example by conduction through the conductive zones, eg. assisted by capillarity due to the material of the zones. The material of the conductive zones can be fibrous or granular or porous solid, e.g. as glass or paper filter material, or gel granules, or a porous membrane possessing pores that communicate in the direction desired for liquid flow. If desired, the swellable material can constitute or be a part of one or both of the conductive zones between which contact is to be made by the swelling effect.

Alternatively contact between two liquid-conductive materials can be broken by the swelling effect. For example, two liquid-conductive materials can be disposed in contact with separating means for forcing them apart by the swelling of the liquid-swellable material.

'Contact' in this context related to two liquid-conductive zones means such contact as will enable liquid to flow or seep or diffuse from one to the other, whether under applied pressure or not: in any case any applied pressure (if present) is to be insufficient to drive liquid flow between the zones when a gap - most often an air gap - is present between them.

In one important embodiment of the invention, contact between liquid-conductive zones is made or formed or enhanced by swelling of a liquid-swellable material and thereby initiates or promotes flow of a washing or reagent liquid which acts to start or stop or modulate a reaction relevant to the test in one of the conductive zones.

For example, the washing/reagent liquid flow can be arranged to stop a chromogenic enzyme reaction occurring in one of the zones, e.g. by altering the aqueous-liquid pH to stop or slow the reaction down to an adequately low rate to allow reasonable time for a user to measure, inspect and/or record the chromogenic result. Alternatively, an auxiliary reagent can be introduced in the liquid flow that results from the making or promoting of contact, to start a detectable reaction or alter its course by effectively substituting one reagent and reaction for another.

The liquid-swellable material can be any solid or gel-form material that is capable of conducting the liquid (usually water or an aqueous medium), which increases substantially in volume when wetted with the liquid, and which does not have any properties which might interfere with the performance of the test or which cannot be removed or neutralised to prevent such interference. An example of a potentially interfering property would be non-specific protein binding, but this can usually be blocked by prior treatment with bovine serum albumin or polyvinylalcohol, for example. Highly-absorbant papers are available commercially and which act as "blotting paper", taking up liquid and expanding in volume to, for example, double their thickness. Other suitable liquid-swellable materials include lyogels. If the gel is granular in form, it can if desired be enclosed within an envelope of liquid-conductive material such as paper, to retain the granular material in situ during manufacture of the test device and storage of the device in the dry state before use.

The invention therefore provides superior test devices and methods for carrying out microchemical tests using liquid samples, wherein a reaction zone in a liquid-conductive zone is exposed to material inflow, e.g. liquid inflow, from at least two different routes and/or at least two different sources, especially for example applied at the same time to the device, arranged so that the reaction zone experiences inflow of liquid from the sources or routes in different proportions and/or at different times during the course of performance of the test.

The composition of the liquid arriving by each of two or more flow paths to the reaction zone can be arranged to be different, either by use of a different liquid source in each case, and/or by use of flow paths which carry or are in contact with or impregnated with a soluble or dispersible material relevant to the test, such as a reagent or diluent or washing material.

The difference in arrival time of two different liquids at the test or reaction zone, and the compositional differences of the liquid as they arrive at the zone, can be selected and adjusted at will from within a wide range to suit the particular requirements of the test or reaction in view.

For example, a competitive binding assay can be carried out by arranging one flow path to bring a sample material and labelled competitor material into contact with a specific binding agent for them both, and for another flow path later to bring in a reagent material to visualise the bound label, after the sample and competitor (in so far as they remain unbound) have been washed out of the reaction zone into an absorbent sink or reservoir by liquid from either flow path or both.

Suitable liquid-conductive materials to compose the flow paths and reaction zone are for example porous, granular and fibrous materials. To supplement the "switching" feature of the invention, a useful method of assuring inflow of liquid from two different routes or sources at two different times into the reaction zone although they may be applied to the test device at the same time, is to use liquid-conductive connecting zones, lines or tracks of different liquid-conductive rate, e.g. achieved by selection of different porosity, fibrous fineness, or other feature affecting liquid conduction.

In general, a test device of the invention incorporates a liquid-conductive region which carries a reagent specific for the test to be carried out, e.g. in immobilised form. Such a reagent can for example be a specific binding reagent such as an antigen or antibody, and can if desired be immobilised on the liquid-conductive carrier by methods well-known per se for adsorbing or coupling such materials to solid carriers, e.g. by physical adsorption to plastics or cellulose-based material or by covalent coupling using per se well-known coupling chemistry, e.g. cyanogen bromide activation of hydroxy (e.g. cellulosic) carriers or modified carriers, or glutaraldehyde or other dialdehyde coupling to amino-group containing carriers or modified carriers.

In use, the devices of the invention can be contacted with sample liquid and/or into washing or reagent liquids, for example by dipping. Most conveniently, the devices can contain in impregnated and/or immobilised form, and possibly in distinct zones all of the reagents required by the test in view, so that no contact with separate reagent liquid is needed.

Further illustration of the invention is given by means of the following drawings, and by the undermentioned details of preparation of materials which can be used in fabricating devices according to the invention. The details given below are not intended to limit the invention.

### Preparations of materials

### A: Selection of liquid-conductive materials:

Representative useful examples of liquid-conductive porous or fibrous sheet materials are as follows, with indications in most cases of pore size, and indications of their relative speed of liquid conduction given as the approximate time taken for aqueous solvent to travel 45 mm in a typical test arrangement:

| Rapidly-conductive materials: | | |
|---|---|---|
| Whatman 3MM | | (about 3 minutes) |
| Whatman 3ET | | (about 1 m 20 sec) |
| Whatman 113 | (30 µm (micron)) | (about 0 m 30 sec) |
| Whatman NO4 | (20 µm (micron)) | (about 1 m 30 sec) |

| Slower-conductive materials: | | |
|---|---|---|
| Whatman NO1 | (10 µm (micron)) | (about 5 m 40 sec) |
| Whatman NO2 | ( 7 µm (micron)) | (about 5 m 30 sec) |
| Whatman N050 | ( 2 µm (micron)) | (about 8 m 40 sec) |
| Whatman nitrocellulose | (1 µm (micron)) | (about 23 minutes) |
| Whatman nitrocellulose | (5 µm (micron)) | (about 19 minutes) |

It is also useful to note that where a particular fibrous sheet has fibres orientated in a predominant axial direction, liquid flow along that axial direction is faster than flow in the transverse direction.

It is mentioned that any of the materials noted as rapidly-conductive" can be used as the slower conductive track if a more rapidly-conductive material is used as the faster track - and the contrary for the materials noted as slow-conductive. Relative speed is more important, though in general of course it is desirable to have a test device working as fast as can be arranged.

### B: Zonal activation and impregnation of liquid-conductive materials:

The materials specified above can be activated for the attachment/immobilisation of specific reagents, especially proteins, to limited zones thereof, by any of many methods well-known per se.

For example, paper filter material, e.g. Whatman 31 ET, can be conveniently activated by immersion in 0.2 M carbonyldiimidazole in pyridine, with gentle stirring, for 1 hour at room temperature, followed by washing with tetrahydrofuran and blown-air drying, for about 20 seconds.

Alternatively, other sheets with rapid chromatographic properties can be used and activated by methods given in the literature, e.g. those mentioned above, or especially Whatman 3MM, Whatman GFF glass fibre filter paper or suitable synthetic membranes, such as Biodyne A (5 µm(micron) pore size) (Trade Marks: available from Whatman and from Pall Corporation respectively).

Liquid-conducting track material with a restricted zone of immobilised protein, especially antibody, can be made for example as follows:- A rectangle of Whatman 31 ET paper measuring about 10 cm in length and 10-100 cm in width is activated as described above, and a reaction zone is formed upon it by applying a stripe of material about 0.5-1 cm wide to the activated paper, the stripe being located about half-way along its 10 cm length and extending throughout its 10-100 cm width. The material can for example be a suitably selected antibody preparation, e.g. 100 microgram/ml in pH 9.5 bicarbonate buffer, e.g. selected anti-beta-(human chorionic gonadotrophin) of affinity K_{A} at least 10⁹, and preferably at least 10¹¹, suitable for immunoassay of human chorionic gonadotrophin using a second (labelled) anti-HCG antibody in a sandwich format. A small volume of such a preparation can be applied using a fine-tipped pipette, fountain pen, air-brush or TLC applicator to give a stripe of applied liquid yielding a reacted layer approximately 0.5-1 cm wide, which will provide a small immunosorbent area in the completed device. When the applied material has reacted with the activated paper for 1 hour at room temperature, excess activated groups are neutralised in ethanolamine for 1 hour and then washed in 0.1M phosphate buffer pH 7 (0.15 M in NaCl) for 1 hour and air-dried at 30°C. Alternatively, the excess activated groups on the paper can be blocked with an inert protein such as BSA or other otherwise inert compound with which the activated groups can react, e.g. polyvinyl alcohol.

Alternatively, Whatman 31 ET material can be activated by contact for 5 hours at room temperature with 0.1M sodium periodate after extensive water-washing. To the activated washed paper, protein is then applied (using the technique described above) in 0.55M borate buffer (pH 8.5, 0.2M in NaCl) and allowed to stand 2 hours at room temperature before contact with a 1 mg/ml solution of sodium borohydride and thorough washing with borate buffer.

The material can then be cut up into numerous strips corresponding to the 10 cm length of the original material, each strip carrying a limited zone of the immobilised antibody to function as an immunosorbent, part-way (e.g. about half-way) along its length. In use, this limited zone then becomes a test reaction zone in which the immunoassay reactions take place.

If desired, before cutting up the paper liquid-conductive material into strips, a further reagent stripe can be applied, in the form of a stripe of enzyme-labelled antibody, e.g. alkaline-phosphatase-conjugated anti-HCG, prepared by mixing 100 parts by volume antibody at 3.3 mg/ml concentration to 100 parts by volume alkaline phosphatase at 10 mg/ml concentration. 5 parts by volume 25% polymeric glutaraldehyde are added to the mixture, and the conjugation reaction allowed to continue for 3 hours at (15°-25°C) ambient temperature. The reaction is stopped by adding 5000 parts by volume buffer (0.05 M Tris-HCl, pH 8, with 50 mg/ml ovalbumin, 0.2 mg/ml magnesium chloride, 0.2% sodium azide and 0.2% merthiolate). Before application to the paper, the stock liquid so obtained is diluted 1/20 in 0.1 M phosphate-buffered saline (pH 7) containing 0.1% Triton QS9 (Trade Mark - obtainable from Rohm and Haas) detergent.

Liquid-conductive strips can be prepared with an impregnation of other reagents: for example enzyme substrate, which in the case of a phosphatase enzyme can conveniently be BCIP substrate at 2 mg/ml in 1 M Tris-HCl at pH 9.8, soaked into Whatman No. 1 or Biodyne A (0.5 µm (micron) pore size) and air-dried at 30°C.

### C: Preparation of liquid-swellable liquid-conductive materials:

Material is used which swells very substantially on addition of water and can be selected from a number of commercially-available alternatives, especially IEF (isoelectric focussing) Wick LKB 1850-911 (from LKB Produkter, Sweden), or lyogel. The isoelectric focussing wick material can expand in height from 1 mm to 2 mm thick on contact with water, i.e. roughly double in size. With some materials, especially IEF wick material, it is possible to soak these in substrate solution and dry before assembly of the device.

### Referring to the drawings:

Figure 1 shows in diagrammatic cross-section a test device according to an embodiment of the invention.

Figures 2a and 2b show in diagrammatic section part of the test device according to Figure 1, before, during and after use respectively.

Figures 3 to 5 show in diagrammatic fragmentary section, stages in the operation of an alternative embodiment of the test device.

The materials prepared as described above can be used in the fabrication of devices as shown schematically in the drawings.

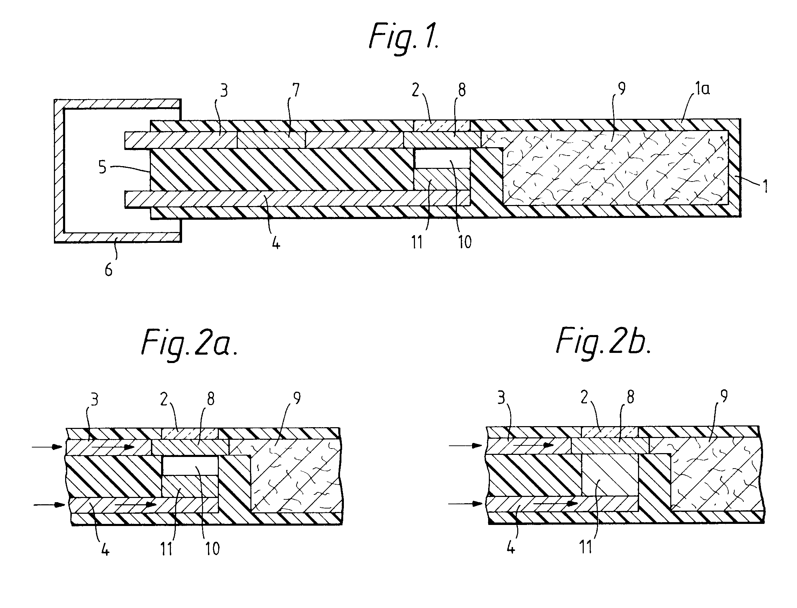

Referring to Figure 1, a test device is shown, suitable for carrying out, for example, an enzyme-linked immunoassay and making a colour-change result visible to the user.

The test device comprises a plastics case 1, preferably opaque or translucent, which also acts as a handling-piece at its end 1a. Case 1 has a transparent viewing position 2, through which the user may see an immunosorbent material as described below.

Passing through casing 1 are two passageways or channels 3 and 4 containing conductive tracks of porous or fibrous liquid-conductive material as described above. The channels or passageways and their respective "fast" and "slow" tracks 3 and 4 are open to contact with the outside world at end 5 of casing 1 opposite handle end 1a. In use, end 5 of casing 1, sensitised by the presence of the ends of liquid conductive tracks 3 and 4, is contacted with sample liquid so that both of tracks 3 and 4 take up the sample liquid.

Any convenient arrangement may be included for contacting sensitised end 5 with sample liquid; for example, a urine collection arrangement 6 as described in EP 0 164 180 may be removably fitted to end 5 of casing 1. Another form of sample collection device is a porous body of absorbant material, e.g. of polymeric material, which can rapidly take up an applied liquid sample and then release it into the two tracks; if desired, the porous sample collector can be provided with a removable moisture-impervious cover.

In the device of Figure 1, track 3 is a relatively fast-conductive track, while track 4 is a relatively slow-conductive track. Track 3 is releasably impregnated at a zone 7 thereof with a conjugate of an enzyme, e.g. alkaline phosphatase, with an antibody, e.g. anti-(human chorionic gonadotrophin), having specificity selected according to the object of the particular test in hand.

A further zone 8 of track 3, farther away from end 5 than zone 7 is, carries covalently-immobilised antibody to confer immunosorbent properties on zone 8 of track 3. Beyond zone 8, track 3 is in liquid-conductive contact with a sink 9 of liquid-absorbent material, e.g. cottonwool, so that sink 9 can take up liquid that arrives by capillary flow after travelling from its point of uptake at end 5. Sink 9 is accommodated in a cavity in end 1a of casing 1.

Behind zone 8 of track 3, a passageway 10 provides communication with track 4, which does not otherwise contact track 3 or sink 9. Before use, there is an air gap, e.g. of the order of up to 1 mm wide, between zone 8 and a "switch" 11 of water-swellable material which is in conductive contact with track 4, and carries (e.g. in an upper layer thereof) a releasable load of impregnated substrate corresponding to the enzyme in zone 7. In use, switch 11 swells by liquid uptake and thereafter makes contact with zone 8.

Use and operation of the device is as follows. The user contacts end 5 with sample liquid. Sample liquid begins to travel along both of tracks 3 and 4, but faster along track 3. Conjugate impregnated at zone 7 is entrained in the liquid flow along track 3. When sample and conjugate reach zone 8 by travel along track 3, immunological binding reactions occur involving the immunosorbent in zone 8, resulting in a variable amount of conjugate becoming bound in zone 8, according to the quantity or concentration of analyte present in the sample liquid, either by "sandwich" complex formation or by competitive binding reaction. Unbound conjugate is washed onwards into sink 9 by continuing flow of sample liquid along track 3. A little later, preferably adjusted by suitable selection of materials and e.g. about 2 minutes later, sample liquid reaches switch 11 after travelling along track 4. This causes switch 11 to swell and make liquid contact with the hinder surface of zone 8 opposite window 2. After this has happened, substrate (e.g. BCIP alkaline phosphatase substrate) from switch 11 flows into zone 8 entrained in liquid supplied by flow along track 4. In zone 8, substrate reacts with bound enzyme according to the quantity of enzyme conjugate immobilised in the binding reaction of the test, and makes a colour change result which is visible through window 2. Continued flow of liquid through tracks 3 and 4 can in certain examples of the device bring about a deceleration or stop to the enzyme reaction in zone 8. Especially advantageously this can be brought about by an advancing front of more acid liquid (e.g. with pH below 7, e.g. in the range 6 or 7) produced by impregnation of part of one of the tracks, e.g. track 4, with suitable buffer, which is entrained in liquid and carried by capillarity to arrive at zone 8 later than the substrate.

Figures 2a and 2b show more clearly the change in configuration of switch 11 during use, as described above.

One of the advantages of this and other embodiments of the invention is that arrangements as described herein can make it unnecessary for the unskilled user, far from sophisticated laboratory facilities, to use analytical washing liquid facilities or even separate liquid water washes: the necessary changes in the liquid environment of the reactive immunosorbent zone can be brought about by the through-flow of sample liquids, e.g. biological or clinical sample liquids including body fluids such as urine.

In a variant part of the device of Figures 1-2, counterpart of switch 11 may be omitted, and passageway 10 may from first to last be filled by liquid-conductive material to allow liquid transfer from slow track 4 to zone 8.

The device of Figure 1 can, for example, be arranged for sandwich immunoassay for an antigen using bound antibody and an enzyme-labelled second antibody. A sampler can be provided to take a liquid sample, mix it with antigen conjugated to detectable label, and apply it as a spot to the upper track. Water can be presented to the free ends of the two tracks, and moves by capillary action through the two tracks. The sample and excess conjugate is washed through the zone of immobilised antibody. In this example the substrate can be presented in the "slow" track and/or in the movable pad of swellable material, in dry form before the device is used, so that it dissolves in the liquids used in the test.

In an alternative arrangement, the conjugate is impregnated on a zone of the fast track and sample is infiltrated in from the left into both tracks, where it functions both as a sample and as wash liquid.

In a further alternative arrangement, the slower track is impregnated with a further reagent so that the wash liquid that infiltrates the carried reagent zone via the slower route, and the swellable filter pad is modified in its composition, e.g. so that it is buffered to a desired acid or alkaline pH that will affect the test reaction, and thereby starts or stops (usually stops) a detection reaction previously taking place in the carried reagent zone.

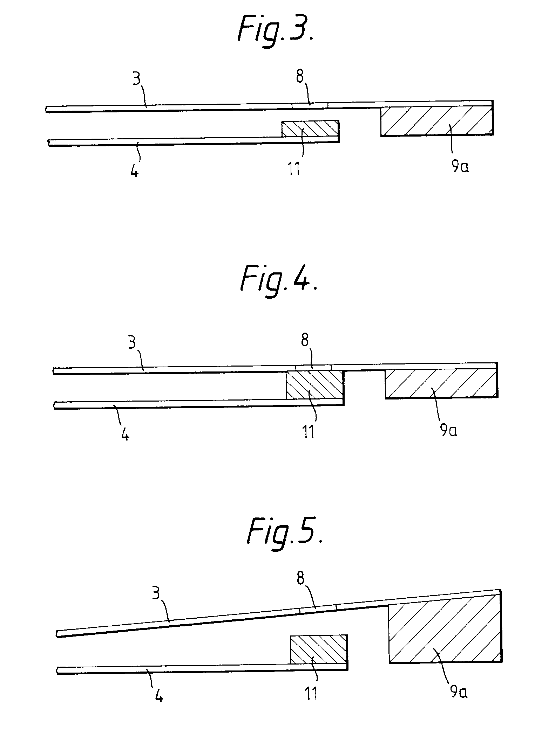

Figures 3, 4 and 5 schematically show a development of the features of Figure 1, in which a further and larger swellable filter pad sink is provided, (in the position of sink 9 in Figure 1), distant to the point of contact established between the tracks by the first swellable pad described in connection with Figure 1, and arranged to force the tracks apart when it swells, so re-establishing the air gap and preventing further liquid transfer from the lower to the upper track. Figure 3 shows the device diagrammatically in its dry state; Figure 4 after an intermediate stage in use when the first swellable pad has become wetted and swollen by reagent, sample or washing liquid and established contact between the lower and upper tracks; and Figure 5 after a final stage in which the second swellable pad has swollen and forced the lower and upper tracks apart again, thereby hindering further liquid flow between them.

## Claims

1. A test device for detecting the presence of an analyte in a liquid sample and incorporating a plurality of liquid-conductive zones (3, 4) which form separate liquid flow paths which deliver separate liquid streams to a region (8) in the test device during the test procedure, characterised in that the device incorporates a liquid-swellable material (11) which is arranged to swell by contact with liquid sample and/or reagent and thereby to enhance or make or break or restrict contact between two liquid-conductive zones, during the course of the test procedure, to control relative liquid flows in the separate flow paths.

2. A device according to claim 1, characterised in that swelling of the liquid-swellable material makes or enhances contact between a first liquid-conductive zone and a second liquid-conductive zone.

3. A device according to claim 2, characterised in that the contact between the first liquid-conductive zone and the second liquid-conductive zone initiates or promotes transfer of a washing and/or reagent liquid between the zones.

4. A device according to claim 1, characterised in that between two liquid-conductive zones is broken by the swelling of the liquid-swellable material.

5. A device according to any one of the preceding claims, containing two separate liquid flow pathways leading to a common detection zone wherein the result of an enzyme-linked immunoassay can be visualised, the first pathway being provided to deliver a liquid sample to the detection zone and the second pathway being connectable with the detection zone only via a liquid-swellable material which becomes swollen during the course of the assay and thereafter is able to deliver to the detection zone an auxiliary reagent which is necessary for the visualisation of the assay result.

## Patentansprüche

1. Untersuchungsvorrichtung zum Nachweis des Vorliegens eines Analyten in einer Flüssigkeitsprobe, umfassend eine Vielzahl flüssigkeitsleitender Zonen (3,4), die getrennte Flüssigkeitsfließwege bilden, die während des Untersuchungsverfahrens getrennte Flüssigkeitsströme in eine Region (8) in der Untersuchungsvorrichtung abgeben, dadurch gekennzeichnet, daß die Vorrichtung ein flüssigkeitsquellfähiges Material (11) enthält, das angeordnet ist, um bei Kontakt mit der Flüssigkeitsprobe und/oder einem Reagenz zu quellen und dadurch während des Untersuchungsverfahrens den Kontakt zwischen zwei flüssigkeitsleitenden Zonen zu verstärken, herbeizuführen, zu unterbrechen oder zu beschränken, um die relativen Flüssigkeitsflüsse in den getrennten Fließwegen zu regulieren.

2. Vorrichtung nach Anspruch 1, dadurch gekennzeichnet, daß das Quellen des flüssigkeitsquellfähigen Materials den Kontakt zwischen einer ersten flüssigkeitsleitenden Zone und einer zweiten flüssigkeitsleitenden Zone herbeiführt oder verstärkt.

3. Vorrichtung nach Anspruch 2, dadurch gekennzeichnet, daß der Kontakt zwischen der ersten flüssigkeitsleitenden Zone und der zweiten flüssigkeitsleitenden Zone die Übertragung einer Wasch- und/oder Reagenzflüssigkeit zwischen den Zonen einleitet oder beschleunigt.

4. Vorrichtung nach Anspruch 1, dadurch gekennzeichnet, daß der Kontakt zwischen zwei flüssigkeitsleitenden Zonen durch das Quellen des flüssigkeitsquellfähigen Materials unterbrochen wird.

5. Vorrichtung nach irgendeinem der vorhergehenden Ansprüche, die zwei getrennte Flüssigkeitsfließwege aufweist, die zu einer gemeinsamen Nachweiszone führen, in welcher das Ergebnis eines enzymverbundenen Immunoassays sichtbar gemacht werden kann, wobei der erste Weg vorgesehen ist, der Nachweisezone eine Flüssigkeitsprobe zuzuführen, und der zweite Weg mit der Nachweiszone nur über ein flüssigkeitsquellfähiges Material verbindbar ist, das während des Assayvorganges quillt und danach in der Lage ist, der Nachweiszone ein Hilfsreagenz zuzuführen, das zur Sichtbarmachung des Assayergebnisses notwendig ist.

## Revendications

1. Dispositif de test pour détecter la présence d'un analyte dans un échantillon de liquide, le dispositif de test incorporant une pluralité de zones de conduction de liquide (3, 4) qui forment des voies d'écoulement de liquide distinctes qui acheminent des jets ou courants de liquide distincts jusqu'à une région (8) située dans le dispositif de test pendant le déroulement de la procédure de test, caractérisé en ce que le dispositif incorpore un matériau augmentant de volume en présence de liquides (11) qui est agencé de manière à augmenter de volume par contact avec un échantillon de liquide et/ou un réactif et pour ainsi augmenter, ou établir, ou rompre, ou limiter le contact entre deux zones de conduction de liquide pendant le déroulement de la procédure de test afin de commander des écoulements de liquide relatifs dans les voies d'écoulement distinctes.

2. Dispositif selon la revendication 1, caractérisé en ce que l'augmentation de volume du matériau augmentant de volume en présence de liquides établit ou augmente le contact entre une première zone de conduction de liquide et une seconde zone de conduction de liquide.

3. Dispositif selon la revendication 2, caractérisé en ce que le contact entre la première zone de conduction de liquide et la seconde zone de conduction de liquide initie ou stimule le transfert d'un liquide laveur et/ou réactif entre les zones.

4. Dispositif selon la revendication 1, caractérisé en ce que le contact entre deux zones de conduction de liquide est rompu par l'augmentation de volume du matériau augmentant de volume en présence de liquides.

5. Dispositif selon l'une quelconque des revendications précédentes, contenant deux voies d'écoulement de liquide distinctes qui conduisent à une zone de détection commune dans laquelle le résultat d'un dosage immuno-enzymatique peut être visualisé, la première voie étant destinée à acheminer un échantillon de liquide jusqu'à la zone de détection et la seconde voie pouvant être reliée à la zone de détection seulement via un matériau augmentant de volume en présence de liquides qui augmente de volume pendant le déroulement du dosage et qui peut ensuite acheminer jusqu'à la zone de détection un réactif auxiliaire qui est nécessaire pour la visualisation du résultat du dosage.
